Europäisches Patentamt

European Patent Office

Office européen des brevets

(11) Publication number: **0 181 300**
**A2**

# (12) EUROPEAN PATENT APPLICATION

(21) Application number: **85850336.0**

(22) Date of filing: **24.10.85**

(51) Int. Cl.⁴: **D 04 H 1/54**

(30) Priority: **08.11.84 SE 8405594**

(43) Date of publication of application:
**14.05.86 Bulletin 86/20**

(84) Designated Contracting States:
**DE FR GB NL**

(71) Applicant: **MÖLNLYCKE AKTIEBOLAG**

**S-405 03 Göteborg(SE)**

(72) Inventor: **Billgren, Thomas**
**01 3560**
**S-43041 Kullavik(SE)**

(72) Inventor: **Forgar, Monica**
**Marklandsgatan 11**
**S-41477 Göteborg(SE)**

(74) Representative: **Berg, Sven Anders et al,**
**H. ALBIHNS PATENTBYRA AB Box 7664**
**S-103 94 Stockholm(SE)**

(54) **Liquid absorbing disposable article.**

(57) A liquid absorbing disposable article such as a medical pad, a drying cloth, a washmitten, a round swab, a diaper, a sanitary napkin or the like, said article at least substantially consisting of a liquid absorbing textile material in the form of a nonwoven textile (fiber fabric) with a weight per unit area of between about 10 and 60 g/m² wherein the fibres incorporated are bonded together and consist of a blend of several different fiber types of which one is at least one high-shrinkage melt fiber present in the textile material in a shrunk state after the exposure to thermal shrinkage, and another one is at least one low-shrinkage fiber remaining intact after exposure to said thermal shrinkage, providing thereby a crimped and highly bulky textile material, the inventive article being distinguished in that the textile material is subjected to thermal shrinkage at spaced intervals in a regular pattern in order to obtain a textile material with an increased bulk and resilience.

STEP I

# LIQUID ABSORBING DISPOSABLE ARTICLE

The present invention relates to a liquid absorbing disposable article such as a compress, a drying cloth, a washmitten, a round swab, a diaper, a sanitary napkin or the like, said article at least substantially consisting of a liquid absorbing textile material.

One of the demands placed on liquid absorbing articles of the above-mentioned type is that the absorbency of the textile material included be adapted to its specific purpose with regard to rate of absorption, while also taking into account the amount of fluid said material is capable to absorb and retain.

Moreover, if the article is to be used for medical purposes, for example in the treatment of wounds and the like, further demands must be added such as freedom from particle release and a comparatively rough surface structure, such a surface structure being preferred both for cleansing purposes when drying off wounds and skin surfaces for example, and for providing friction in medical dressings, the pads forming part thereof then being more easily retained in their intended positions due to the high friction towards their environment.

When using the product as a drying cloth the material therein must, beyond having a sufficient rate and capacity of absorption, also be bulky and resilient enough in a wet state while simultaneously affording an acceptable wet breaking strength; these factors also being applicable for washmittens for example.

In the past, products of this type were manufactured from conventional textile, and gauze for example was used in wound compresses and so-called round swabs for the cleansing of wounds, whereas terry fabric, for example, was used for washmittens.

The development within this field has however led to the increased use of disposable articles, and for this reason the afore-mentioned prior art textile material has also to a large extend come to be replaced by less expensive textile materials in the form of so-called nonwoven textile (fiber fabric), the complicated textile manufacturing technique in its entirety being replaced by high-production units. In order to create resemblance with conventional materials, so-called high shrinkage nonwoven material had already been developed, that is a textile material composed of a nonwoven textile having a weight per unit area of between about 10 and 60 $g/m^2$, the bonded-together fiber in said material being composed of a blend of several different fiber types one of which being a hydrophobic high-shrinkage melt fiber appearing in the textile material in a shrunk state after the exposure to shrinkage temperature, and another a hydrophilic, low-shrinkage cellulose-based fiber remaining unaffected by said shrinkage temperature, providing thereby a high bulk and crimp to the textile material. Such a textile material is described in Applicant's earlier Swedish patent published under No. 423 034 (Laid-Open Publication No. 7809785-4). This material contains a proportion of hydrophobic high-shrinkage melt fiber of between 10 and 50%; preferably however a proportion of between 25 and 35% of the fiber type composition. Said material has been produced by heating with a uniform hot air stream the preferably print-bonded fiber fabric above the shrinkage temperature for the melt fiber, accomplishing in this way a shrinkage in the order of between 10 and 40% in the longitudinal direction of the fiber, all in accordance with the selected type of melt fiber.

The object of the present invention is to provide an article of the kind mentioned in the introduction which, like the product defined above, contains a textile material of the high-shrinkage nonwoven type but which, in contrast thereto, exhibits an improved bulk and resilience while being if not superior to, at least comparable to the previously known material with regard to wet breaking strength, liquid absorbency, material consumption etc. Tests with liquid absorbing products carried out according to the invention have proved that this object is achieved by the fibers included in the inventive material constituting an admixture of several different types of fibers of which one is at least one high-shrinkage melt fiber present in the textile material in a shrunk state due to heat treatment at shrinkage temperature, and another one is at least one low-shrinkage fiber remaining unaffected by said shrinkage temperature, and that the textile material is treated by thermal shrinkage at spaced intervals in a preferably regular pattern, the textile material in this manner exhibiting an increased bulk and elasticity.

The fibrous web used as a basis when manufacturing the inventive textile material is made cohesive by print-bonding the fibers in the material into a basic pattern. The design of this pattern is of vital importance for the appearance of the material subsequent to shrinkage. The shrinkage of the fiber web reaches its maximum within the non-bonded areas. For example, in a double-diagonal base pattern the fiber shrinkage is most pronounced between the bonding lines, which will give rise to the formation of elevated "cushions" in the material. In order for the material, after exposure to thermal shrinkage at spaced intervals, to obtain its optimum bulk and elasticity, the amount of high shrinkage fibers included therein must definitely constitute an essential part of the total quantity of fibers and should, for example in so-called melt-bonding and with high shrinkage fibers serving also as melt fibers, lie between about 65% and about 70%. In this manner there is achieved with melt-bonding a satisfactory bonding effect for each individual fiber without the risk of fiber release. Thus, the textile material can be melt-bonded without the use of a specific binder, and the melt fibers are fused together along the lines of the print-bonded base pattern. In melt-bonding, this base pattern is preferably accomplished by calendering the fiber material between two hot rolls of which one is smooth while the other has a surface structure corresponding to the aforesaid pattern, the fibers thereby melting together along the lines of the pattern for bonding the fibers into a nonwoven textile material. To advantage, the fibers in this material have a length in the order of 40-55 mm, which contributes to reducing the risk of fiber release in the final product. The fibers in the textile material are held together in base patterns which can be formed to square shape, for example by intersecting diagonals, or in a so-called mini-track pattern having sections of its pattern located along intersecting diagonals as well but constituting short sections of which single portions extending along one diagonal intersect other diagonals in the gap between individual, short pattern portions.

The thermal shrinkage treatment takes place at a temperature lying immediately below the melting point of the melt fiber and may, for example in the treatment of certain fiber types such as polypropylene fibers, lie between about 160 and 170$^{\circ}$C while occupying a period of two seconds. However, said values are in no way crucial. To advantage, the thermal treatment can be carried out along parallel lines. The material bonded to a base pattern is subjected to shrinkage treatment for example by allowing hot air to pass through perforated plates between which the web of material is fed, said plates having rows of holes along which the material is forced to shrink in order for the specific new pattern to be formed. The hot air treatment could also be performed with the aid of a number of hot air jets, for example, directed towards the web of material. Heating is done for example by radiation of hot air, by IR heat radiation, or in any other way.

According to the invention, fibers of polypropylene, polyester, acrylic or the like can be used as high shrinkage melt fibers as well as fibers of a composite material such as polypropylene fibers covered with a polyester coating.

Examples of hydrophilic, low shrinkage fibers are pure cellulose fibers such as cotton, pulp fibers or viscose fibers but also fibers of flax, jute, hemp, cupro and modal, for example. Viscose fibers are used to advantage, that is fibers of regenerated cellulose, or cotton fibers, or a blend of said two hydrophilic fiber materials.

In addition, a separate reinforcing effect of the wet bulk, i.e. the three-dimensional structure of the ready-shrunk material, can be obtained by incorporating in the fiber composition a third fiber to a proportion of up to about 20%. Examples of such a third fiber are thermo-stabilized qualities of polypropylene, polyester and acrylic which take no active part in the actual liquid absorption process and which also remain unaffected by the shrinkage temperature but instead retain their length and resilience subsequent to shrinkage, enabling them as a result to stretchingly influence the hydrophilic fiber or fibers included in the material; reinforcing in this way the ready-shrunk material bulk both with regard to its volume and to its capacity of remaining intact even after thorough wetting.

The novel product presents an enhanced bulkiness and elasticity with the same amount of material as that used in the previously known "shrink nonwoven" material. This increase in bulkiness and resilience is an extremely important factor when using the material as a drying cloth, for medical pads or so-called round swabs. When cleansing wounds with compresses or round swabs, the drying material is gripped with hard pliers and in this connection it is most vital for the material to be resilient enough so as not to harm the wound with the pliers, or to avoid the feel of the pliers through the material.

Despite the fact that in melt-bonding with hydrophobic melt fibers for example, the proportion of hydrophobic melt fibers in the novel material is as high as 60-75% thereof, so that when thermally treated along separate lines in the direction of feed of the manufacturing web it could be referred to as a seersucker material, a surprisingly good absorbency is obtained in the order of 6-7 times its own weight, which even surpasses the capacity of the conventional "shrink nonwoven" material having a substantially higher proportion of hydrophilic fibers than has the novel material according to the invention. In addition to increased elasticity there is thus also obtained a surprisingly good liquid retaining capacity.

The novel material has been described above in connection with products for the treatment of wounds, although many other fields of use are also feasible. In disposable absorption articles such as diapers and sanitary napkins, the novel material is extremely useful for providing an insulating layer facing the body of the wearer. Due to its large volume, the material can form an insulating layer between a saturated absorption core in a diaper or a sanitary napkin and the outer layer facing the body of the user. The material may of course also serve as such an outer layer provided that all fibers included in the material are hydrophobic. This insulating layer would then afford a drier product contacting the body of the user.

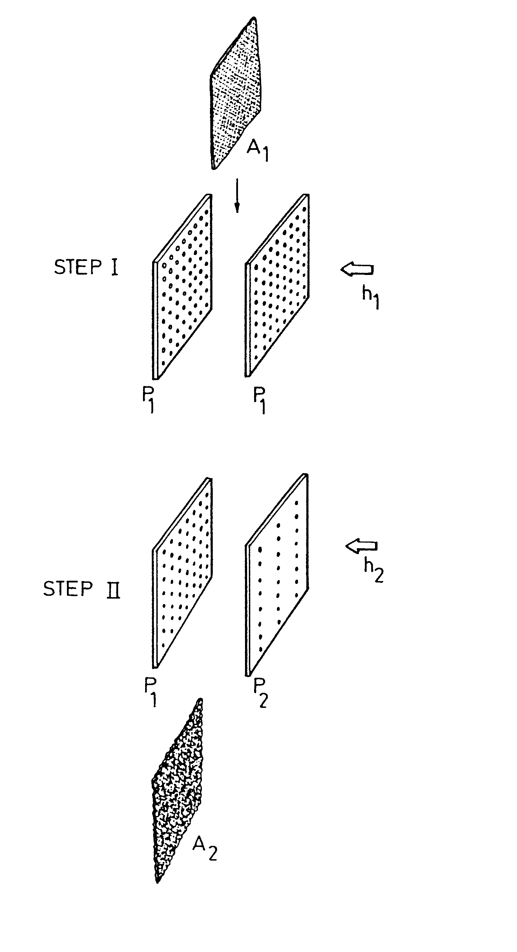

The invention will be described in more detail below with reference to the accompanying drawing figure, illustrating schematically an example of a manufacturing process for an article made according to the invention. The melt-bonded fibrous web, which is print-bonded after calendering (shown cut out at $A_1$), is advanced in the direction of the vertical arrow and is first subjected to preheating between two substantially parallel plates $P_1$ in a step I at a temperature $h_1$ lying below the shrinkage temperature of the melt fiber. The web of material is then fed to a step II for the actual shrinkage process, the material thereby passing between plates $P_1$ and $P_2$. In this stage the plate $P_1$ may be similar to the plates of step I thus emitting radiation at a temperature lying below the shrinkage temperature, whereas the plate $P_2$ has solely one row of holes emitting radiation of higher temperature than the  shrinkage temperatures for the shrinkable fibers in the material, said fibers being forced to shrink along spaced straight paths right ahead of corresponding holes in plate $P_2$ for creating the seersucker pattern. Hot air radiation or for example IR heat radiation is directed substantially at right angles to the plates. The shrink-treated material leaves the process in the form of a web (shown cut out at $A_2$) and from this web-shaped material thus produced, various preselected articles according to the invention can be manufactured such as medical pads, drying cloths, diapers, sanitary napkins etc. in a manner known per se.

The invention is not restricted to the exemplary embodiments set forth above and illustrated in the drawing, but can be modified in numerous ways within the scope of the claims.

The shrinkage need of course not be performed in a pattern constituting spaced straight paths. For example, portions of the web of material separated in the manufacturing direction can be mechanically caught in order to prevent shrinkage along these portions. By the appropriate selection of the geometrical shape of such portions along which shrinkage is inhibited, the final form and absorbtion properties of the material can be varied.

Shrinkage in a regular pattern could further be performed by means of jets being blown to produce shrink heating both longitudinally and transversely to the direction of feed of the web of material.

Bonding in the base pattern may take place otherwise than by melt-bonding. Nonwoven textiles can be manufactured according to the so-called spun-lace method, which means that the fibers are united by means of thin water jets under high pressure and without the use of bonding agents.

In this manner the percentage of hydrophilic fibers in the material can be considerably higher than in melt-bonding, which in turn results in an increased liquid retaining capacity.

CLAIMS

1.     A liquid-absorbing disposable article such as a medical compress, a drying cloth, a washmitten, a round swab, a diaper, a sanitary napkin or the like consisting at least substantially of a liquid absorbing textile material formed of a nonwoven textile (fiber fabric) with a weight per unit area of between approx. 10 and 60 $g/m^2$, the fibers in the material being held together and composed of a blend of several different fiber types of which one is at least one high-shrinkage melt fiber present in the textile material in a shrunk state due to heat treatment at shrinkage temperature whereas another one is at least one low-shrinkage fiber remaining unaffected by said shrinkage temperature, providing thereby a crimped and highly bulky textile material, characterized in that the textile material is treated by thermal shrinkage at spaced intervals in a preferably regular pattern in order to increase the bulk and resilience of the textile material.

2.     An article according to Claim 1, characterized in that the article consists of a melt-bonded material, and that the proportion of high-shrinkage melt fiber is between 60 and approx. 75%, whereas the proportion of low-shrinkage fiber is between approx. 25 and approx. 40% of the blend composed of the two fiber types.

3.     An article according to Claim 2, characterized in that the high-shrinkage melt fiber constitutes between approx. 65 and approx. 70% and the low-shrinkage fiber between approx. 30 and approx. 35% of the blend composed of the two fiber types.

4.     An article according to Claim 1 or 2, characterized in that the textile material is treated by thermal shrinkage along substantially straight spaced lines or the like.

5.     An article according to any one of Claims 1-3, characterized in that the textile material is subjected to thermal shrinkage at spaced portions along substantially parallel lines.

6.	An article according to any one of Claims 1-5, characterized in that the fibers included in the textile material are held together by melt-bonding in a square-shaped pattern.

7.	An article according to any one of Claims 1-5, characterized in that the fibers included in the textile material are held together by melt-bonding in a so-called mini-track pattern.

$A_1$

STEP I

$P_1$ $P_1$

$h_1$

STEP II

$P_1$ $P_2$

$h_2$

$A_2$